# EUROPEAN PATENT APPLICATION

(11) **EP 4 775 563 A1**
(43) Date of publication of application: **15.07.2026**
(21) Application number: 25222764.0
(22) Date of filing: 11.12.2025
(51) Int. Cl.: C07C 39/16, C07C 37/05

(54) **ALKYL BISPHENOL COMPOSITION AND METHOD FOR PREPARING THE SAME**

(30) Priority: 31.12.2024 CN 202411988816
(71) Applicant: Industrial Technology Research Institute, 310401 Hsinchu (TW)
(72) Inventor: CHEN, Chang-An, Hsinchu City (TW); WU, Yi-Cang, Hsinchu City (TW); HUNG, Chien-Yi, Hsinchu City (TW); KUO, Shin-Liang, Hsinchu City (TW)
(74) Representative: Fuchs Patentanwälte Partnerschaft mbB

(57) **Abstract**

An alkyl bisphenol composition and a method for preparing the same are provided. The alkyl bisphenol composition includes 99.85wt% to 99.999wt% of an alkyl bisphenol; 0.0001wt% to 0.01wt% of a monophenolic compound; 0.0001wt% to 0.01wt% of a reaction adduct of alkyl bisphenol; and 0.0001wt% to 0.1497wt% of an aromatic compound, wherein the aromatic compound is not the alkyl bisphenol, the monophenolic compound, or the reaction adduct of alkyl bisphenol.

## Description

The disclosure relates to an alkyl bisphenol composition and a method for preparing an alkyl bisphenol composition.

Conventional chemical processes for recycling polycarbonate (PC) often result in residual impurities that originate from the original material, long-term use and degradation, or are formed during the recycling process. Those impurities will remain in the converted monomer composition, regardless of whether the source material is post-industrial recycled (PIR) or post-consumer recycled (PCR) polycarbonate. It should be noted that the presence of these impurities directly affects the quality of the regenerated polycarbonate (PC), limiting its potential for circular use. For example, if a recovered monomer composition contains impurities such as monophenolic compounds, the monophenolic compounds may interfere with polymerization during repolymerization due to uncontrollable end-capping effects, ultimately affecting the properties of the regenerated polymer. In addition, impurities such as flame retardants, dyes, UV absorbers, mold release agents, or antioxidants not only affect the color performance but also alter the properties of regenerated polycarbonate.

To improve the quality of regenerated polycarbonate, conventional chemical recycling processes often involve additional purification steps such as filtration, washing, ion exchange, adsorption, distillation, and recrystallization for removing impurities from the crude monomer composition. However, these complex processes not only reduce the overall recycling efficiency and increase processing costs but still fail to ensure that the regenerated polycarbonate meets the desired quality standards.

Accordingly, a novel polycarbonate recycling method capable of producing high-purity monomer compositions is called for to solve the aforementioned problems.

The disclosure provides an alkyl bisphenol composition and a method for preparing the same. According to embodiments of the disclosure, the alkyl bisphenol composition includes 99.85wt% to 99.999wt% of an alkyl bisphenol; 0.0001wt% to 0.01 wt% of a monophenolic compound; 0.0001wt% to 0.01wt% of a reaction adduct of alkyl bisphenol; and, 0.0001wt% to 0.1497wt% of aromatic compound, wherein the aromatic compound is not the alkyl bisphenol, the monophenolic compound, or the reaction adduct of alkyl bisphenol.

The disclosure also provides a method for preparing an alkyl bisphenol composition including the following steps. A polycarbonate waste is subjected to a depolymerization process to obtain a crude alkyl bisphenol product. The crude alkyl bisphenol product is subjected to an extraction process in the presence of a first solvent, a second solvent and water (a third solvent) to obtain a two-layer solution which contains a first solution and a second solution, wherein the first solution includes the first solvent, and the second solution includes the second solvent, water, and alkyl bisphenol, wherein the first solvent is not miscible with water, and the second solvent is miscible with water. The second solvent is removed from the second solution to precipitate the alkyl bisphenol composition of the disclosure from the second solution.

A detailed description is given in the following embodiments.

The present disclosure can be more fully understood by reading the subsequent detailed description and examples with references made to the accompanying drawings, wherein:

Figure is a flow chart illustrating the method for preparing the alkyl bisphenol composition according to an embodiment of the disclosure.

The alkyl bisphenol composition and the method for preparing an alkyl bisphenol composition are described in detail in the following description. In the following detailed description, for purposes of explanation, numerous specific details and embodiments are set forth in order to provide a thorough understanding of the present disclosure. The specific elements and configurations described in the following detailed description are set forth in order to clearly describe the present disclosure. It will be apparent, however, that the exemplary embodiments set forth herein are used merely for the purpose of illustration, and the inventive concept may be embodied in various forms without being limited to those exemplary embodiments. As used herein, the term "about" in quantitative terms refers to plus or minus an amount that is general and reasonable to persons skilled in the art.

Furthermore, the use of ordinal terms such as "first", "second", "third", etc., in the disclosure to modify an element does not by itself connote any priority, precedence, order of one claim element over another or the temporal order in which it is formed, but are used merely as labels to distinguish one claim element having a certain name from another element having the same name (but for use of the ordinal term) to distinguish the claim elements.

The disclosure provides an alkyl bisphenol composition and a method for preparing the same. In the alkyl bisphenol composition used for regenerating polycarbonate, the presence of impurities (such as monophenolic compounds, incomplete depolymerization products (i.e., reaction adducts of alkyl bisphenol), additives or their derivatives) may affect the properties of the regenerated polycarbonate (PC), such as the degree of polymerization, final molecular weight, color specifications, and stability, thereby limiting the applicability of the final product. Therefore, it is important to reduce the impurity content in the alkyl bisphenol composition recovered from polycarbonate. Due to structural characteristics, many additives containing luminescent groups (such as flame retardants, dyes, UV absorbers, mold release agents, or antioxidants) exhibit strong intermolecular interactions with the aromatic ring structure of alkyl bisphenol, making it difficult to effectively and completely remove these impurities through simple adsorption, washing, or precipitation processes. Furthermore, even when high-temperature distillation is used to purify the recovered alkyl bisphenol composition, the impurities in the composition may undergo side reactions with alkyl bisphenol at high temperature, potentially leading to the formation of additional impurities. Accordingly, the disclosure provides a method for preparing an alkyl bisphenol composition, in which a specific multi-solvent extraction process is employed to significantly reduce the impurity content of the alkyl bisphenol composition without compromising the recovery rate. This process yields a high-purity (purity ≥ 99.85%) and low-APHA color (≤ 80) regenerated alkyl bisphenol composition, thereby enhancing the quality of regenerated polycarbonate products.

According to embodiments of the disclosure, the alkyl bisphenol composition includes 99.85wt% to 99.999wt% (such as 99.86wt%, 99.87wt%, 99.88wt%, 99.89wt%, 99.9wt%, 99.91wt%, 99.92wt%, 99.93wt%, 99.94wt%, 99.95wt%, 99.96wt%, 99.97wt%, 99.98wt%, 99.99wt%, 99.991wt%, 99.992wt%, 99.993wt%, 99.994wt%, 99.995wt%, 99.996wt%, 99.997wt%, or 99.998wt%) of an alkyl bisphenol; 0.0001wt% to 0.01wt% (such as 0.0003wt%, 0.0005wt%, 0.001wt%, 0.0015wt%, 0.002wt%, 0.0025wt%, 0.003wt%, 0.0035wt%, 0.004wt%, 0.0045wt%, 0.005wt%, 0.0055wt%, 0.006wt%, 0.0065wt%, 0.007wt%, 0.0075wt%, 0.008wt%, 0.0085wt%, 0.009wt%, or 0.0095wt%) of a monophenolic compound; 0.0001wt% to 0.01wt% (such as 0.0003wt%, 0.0005wt%, 0.001wt%, 0.0015wt%, 0.002wt%, 0.0025wt%, 0.003wt%, 0.0035wt%, 0.004wt%, 0.0045wt%, 0.005wt%, 0.0055wt%, 0.006wt%, 0.0065wt%, 0.007wt%, 0.0075wt%, 0.008wt%, 0.0085wt%, 0.009wt%, or 0.0095wt%) of a reaction adduct of alkyl bisphenol; and, 0.0001wt% to 0.1497wt% (such as 0.0001wt%, 0.0005wt%, 0.001wt%, 0.002wt%, 0.005wt%, 0.01wt%, 0.02wt%, 0.03wt%, 0.04wt%, 0.05wt%, 0.06wt%, 0.07wt%, 0.08wt%, 0.09wt%, 0.1wt%, 0.11wt%, 0.12wt%, 0.13wt%, 0.14wt%, 0.145wt%, 0.148wt%, or 0.149wt%) of an aromatic compound, wherein the aromatic compound is not the alkyl bisphenol, the monophenolic compound, or the reaction adduct of alkyl bisphenol. Namely, the impurities of the alkyl bisphenol composition of the disclosure (i.e., components other than alkyl bisphenol) may be less than 0.15 wt% (such as less than 0.14 wt%, less than 0.13 wt%, less than 0.12 wt%, less than 0.11 wt%, less than 0.10 wt%, less than 0.09 wt%, less than 0.08 wt%, less than 0.07 wt%, less than 0.06 wt%, less than 0.05 wt%, less than 0.04 wt%, less than 0.03 wt%, less than 0.02 wt%, or less than 0.01 wt%). According to embodiments of the disclosure, the components of the alkyl bisphenol composition are analyzed using high performance liquid chromatography (HPLC).

In the alkyl bisphenol composition, if the content of monophenolic compound is high, the degree of polymerization and the properties of the regenerated polycarbonate would be deteriorated due to the uncontrollable end-capping effects. In addition, if the residual amount of the reaction adduct of alkyl bisphenol or the aromatic compound is high, it would not only affect the color performance but also further alter the properties of the regenerated polycarbonate.

According to embodiments of the disclosure, the alkyl bisphenol may have a structure represented by Formula (I): 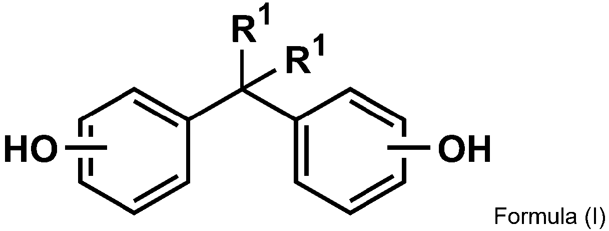 , wherein each R¹ is independently C1-C4 alkyl group. According to embodiments of the disclosure, the alkyl bisphenol may be

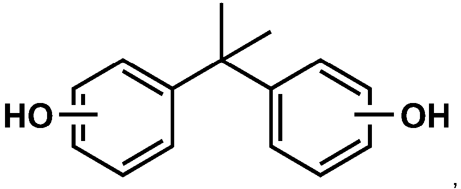

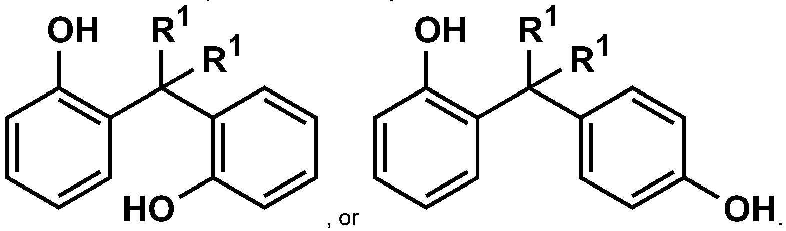

According to embodiments of the disclosure, the monophenolic compound may be phenol, cresol, xylenol, or a combination thereof.

According to embodiments of the disclosure, the reaction adduct of alkyl bisphenol may be an incompletely depolymerized product formed during the depolymerization of polycarbonate, such as a condensation product of alkyl bisphenol and alkyl carbonate ester.

According to embodiments of the disclosure, the reaction adduct of alkyl bisphenol may have a structure represented by Formula (II) 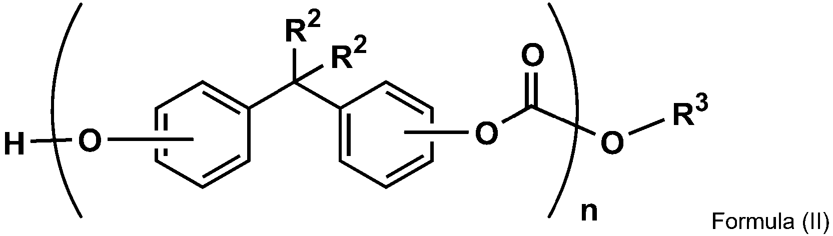 , wherein R² and R³ are independently C1-C4 alkyl group; and, 1,000≥n≥1.

According to embodiments of the disclosure, the above-mentioned C1-C4 alkyl group may be a linear or branched alkyl group. For example, C1-C4 alkyl group may be methyl, ethyl, n-propyl, isopropyl, n-butyl, sec-butyl, iso-butyl, or tert-butyl

According to embodiments of the disclosure, the aromatic compound is not the alkyl bisphenol, the monophenolic compound, or the reaction adduct of alkyl bisphenol. According to embodiments of the disclosure, the aromatic compound may be an additive used in polycarbonate products or an impurity derived therefrom, wherein the additive may be a flame retardant, dye, UV absorber, mold release agent, or antioxidant. According to embodiments of the disclosure, the impurity derived from the additive refers to an additive reaction adduct formed as a result of side reactions during the preparation of the alkyl bisphenol composition.

According to embodiments of the disclosure, the aromatic compound may be an anthraquinone compound, an azo compound, a benzotriazole compound, a benzopyran compound, or a combination thereof. According to embodiments of the disclosure, the anthraquinone compound may have a structure represented by Formula (III) 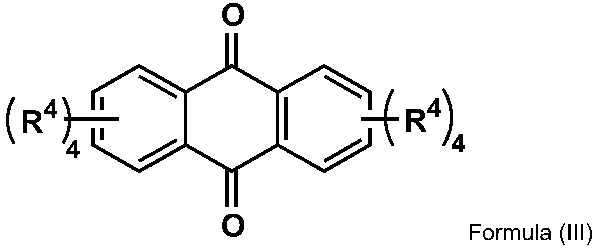 , wherein R⁴ is independently hydrogen, C1-C4 alkyl group, or amine group. For example, the anthraquinone compound may be 2-methylanthraquinone, 2-ethylanthraquinone, 1,3-dimethylanthraquinone, 2,3-dimethylanthraquinone, 1,4-dimethylanthraquinone, 2,7-dimethylanthraquinone, 2-n-propylanthraquinone, 2-isopropylanthraquinone, 2-sec-butylanthraquinone, 2-tert-butylanthraquinone, 2-sec-pentylanthraquinone, 2-tert-pentylanthraquinone, 2-aminoanthraquinone, 1,4-diaminoanthraquinone, 1,5-diaminoanthraquinone, 2,6-diaminoanthraquinone, or a combination thereof. According to embodiments of the disclosure, the azo compound may be an azo dye. According to embodiments of the disclosure, the benzotriazole compound may be 2-(2'-hydroxy-5'-methylphenyl)benzotriazole, 2-(2'-hydroxy-3',5'-di-tert-butylphenyl)benzotriazole, 2-(2'-hydroxy-3',5'-di-tert-butylphenyl)-5-chlorobenzotriazole, 2-(2'-hydroxy-3'-tert-butyl-5'-methylphenyl)-5-chlorobenzotriazole, 2-(2'-hydroxy-5'-tert-octylphenyl)-2H-benzotriazole, 2-(2'-hydroxy-5'-tert-butylphenyl)benzotriazole, 2-(2'-hydroxy-5'-methacryloxyphenyl)-2H-benzotriazole, 2-(2'-hydroxy-3',5'-di-tert-pentylphenyl)benzotriazole, 2-[2'-hydroxy-3',5'-bis(α,α-dimethylbenzyl)phenyl]-2H-benzotriazole, 2-[2'-hydroxy-3'-(3",4",5'',6"-tetrahydrophthalimidomethyl)-5'-methylphenyl]benzotriazole, 2,2'-methylenebis[4-(1,1,3,3-tetramethylbutyl)-6-(2H-benzotriazol-2-yl)phenol], or a combination thereof. According to embodiments of the disclosure, the benzopyrone compound may be a benzopyrone dye.

According to embodiments of the disclosure, the above-mentioned C1-C4 alkyl group may be a linear or branched alkyl group. For example, C1-C4 alkyl group may be methyl, ethyl, n-propyl, isopropyl, n-butyl, sec-butyl, iso-butyl, or tert-butyl.

According to embodiments of the disclosure, the alkyl bisphenol composition may have an APHA color less than or equal to 80, such as 1, 2, 5, 10, 15, 20, 25, 30, 35, 40, 45, 50, 55, 60, 65, 70, or 75. According to embodiments of the disclosure, the APHA color of the alkyl bisphenol composition may range from 1 to 80. According to embodiments of the disclosure, the APHA color is measured in accordance with the method specified in ASTM D1209-05, wherein the test sample (5g) is dissolved in methanol (7ml) prior to measurement.

According to embodiments of the disclosure, the disclosure also provides a method for preparing an alkyl bisphenol composition, such as a method for preparing an alkyl bisphenol composition from a polycarbonate waste. As shown in Figure, the method for preparing the alkyl bisphenol composition 10 may include the following steps. First, a polycarbonate waste is subjected to a depolymerization process to obtain a crude alkyl bisphenol product (step 12), wherein the crude alkyl bisphenol product includes alkyl bisphenol (i.e. the main product obtained after the depolymerization of polycarbonate). Next, the crude alkyl bisphenol product is subjected to an extraction process in the presence of a first solvent, a second solvent and water (a third solvent) to obtain a two-layer solution which contains a first solution and a second solution (step 14), wherein the first solution includes the first solvent; the second solution includes the second solvent, water, and alkyl bisphenol (i.e. the alkyl bisphenol is extracted by the second solvent and water in the extraction process). Next, the second solvent is removed from the second solution to precipitate the alkyl bisphenol composition of the disclosure from the second solution (step 16) (the alkyl bisphenol composition exhibits poor solubility in water). It should be noted that, in comparison with conventional two-solvent extraction processes, the method for preparing the alkyl bisphenol composition described in the disclosure utilizes a multi-solvent (i.e., first solvent, second solvent, and water) extraction process to extract alkyl bisphenol. This approach not only increases the recovery rate (overcoming the issue of significant alkyl bisphenol loss in the conventional extraction processes) but also reduces the impurity content in the resulting alkyl bisphenol composition, thereby enhancing its purity.

According to embodiments of the disclosure, the crude alkyl bisphenol product may be a mixture (also called mixture containing alkyl bisphenol (including solvent)) obtained after performing a depolymerization process on polycarbonate waste. In addition, the crude alkyl bisphenol product may also be the result (excluding solvent) obtained after performing a depolymerization process on polycarbonate waste followed by a concentration step. According to embodiments of the disclosure, the order of adding the first solvent, second solvent, and water during the extraction process does not affect the extraction result. For example, the first solvent and second solvent may be mixed with the crude alkyl bisphenol product first, followed by the addition of water for the extraction process. Alternatively, the second solvent and water may be mixed with the crude alkyl bisphenol product first, followed by the addition of the first solvent. Furthermore, the second solvent may be mixed with the crude alkyl bisphenol product first, followed by the addition of the first solvent and water for the extraction process.

According to embodiments of the disclosure, the polycarbonate waste may be polycarbonate bottle flakes, polycarbonate films, polycarbonate light diffusers, polycarbonate automotive lamp housings, polycarbonate wafer carriers, or a combination thereof. According to embodiments of the disclosure, the polycarbonate waste may be made from a composition including additives and polycarbonate resin, wherein the additive may be a flame retardant, dye, UV absorber, mold release agent, or antioxidant.

According to embodiments of the disclosure, the polycarbonate waste is subjected to a depolymerization process in the presence of a depolymerization composition, wherein the depolymerization composition includes C1-C6 aliphatic monoalcohol, and the process temperature of the depolymerization process may be 50°C to 80°C, and the process time of the depolymerization process may be 1 to 12 hours. According to embodiments of the disclosure, when the upper limit of the process temperature of depolymerization is controlled at 80°C, it can prevent the formation of additional impurities caused by side reactions between the alkyl bisphenol (product) or additives in the polycarbonate waste during depolymerization. On the other hand, when the lower limit of the depolymerization process temperature is controlled below 50°C, the insufficient energy provided may fail to overcome the activation energy of the depolymerization reaction, resulting in a lower yield. According to embodiments of the disclosure, C1-C6 aliphatic monoalcohol may be methanol, ethanol, n-propanol, isopropanol, n-butanol, isobutanol, sec-butanol, tert-butanol, n-pentanol, isopentanol, n-hexanol, or a combination thereof. According to embodiments of the disclosure, the depolymerization composition further includes a co-solvent, wherein the co-solvent may be dichloromethane, chloroform, carbon tetrachloride, cyclohexanone, methyl ethyl ketone (MEK), acetone, toluene, xylene, dimethyl carbonate, diethyl carbonate, or a combination thereof. According to embodiments of the disclosure, the depolymerization composition may further include a catalyst, wherein the catalyst is sodium hydroxide, potassium hydroxide, or a combination thereof. According to embodiments of the disclosure, the amount of C1-C6 aliphatic monoalcohol, co-solvent , and catalyst is not limited and may be optionally modified by a person of ordinary skill in the field. For example, the weight ratio of the polycarbonate waste to the C1-C6 aliphatic monoalcohol may be from 2:1 to 1:5; the weight ratio of the C1-C6 aliphatic monoalcohol to the co-solvent may be from 1:9 to 9:1. The weight ratio of the catalyst to the polycarbonate waste may be from 0.5:100 to 5:100.

According to embodiments of the disclosure, before subjecting the crude alkyl bisphenol product to the extraction process, the C1-C6 aliphatic monoalcohol, co-solvent, and catalyst of the crude alkyl bisphenol product may be removed first.

It should be noted that, in the extraction process, the first solvent is a solvent that is less likely to dissolve alkyl bisphenol, while the second solvent is a solvent that more easily dissolves alkyl bisphenol. Namely, in comparison with the first solvent, alkyl bisphenol is more inclined to dissolve in the second solvent. Since alkyl bisphenol dissolves more easily in the second solvent and water, after the extraction process, alkyl bisphenol will be extracted by the second solvent and water, resulting in the concentration of alkyl bisphenol in the second solution being much greater than in the first solution. Additionally, the main solvent in the first solution is the first solvent (i.e., the amount of the first solvent in the first solution is greater than that of water and the second solvent in the first solution). The main solvent in the second solution is the second solvent and water (i.e., the amount of the second solvent and water in the second solution is greater than the amount of the first solvent). According to embodiments of the disclosure, the first solvent is immiscible with water, while the second solvent is miscible with water.

According to embodiments of the disclosure, the solubility of the alkyl bisphenol in the first solvent at 30°C is less than or equal to 2wt% (such as less than or equal to 1.5wt%, or less than or equal to 1wt%), and the solubility of the alkyl bisphenol in the second solvent at 30°C is greater than or equal to 5wt% (such as greater than or equal to 6wt%, greater than or equal to 7wt%, or greater than or equal to 8wt%). The method for measuring the solubility of alkyl bisphenol in a solvent includes the following steps. Alkyl bisphenol is added to the solvent at 30°C (where the weight ratio of alkyl bisphenol to solvent is 1:1). After stirring for 10 minutes and standing for 1 hour, the weight of the undissolved alkyl bisphenol is measured, and the weight percentage (wt%) of alkyl bisphenol dissolved in the solvent is determined. According to embodiments of the disclosure, the saturated vapor pressure of the second solvent is greater than that of water at a temperature between 25°C and 70°C. As a result, the second solvent is more easily removed relative to water.

According to embodiments of the disclosure, the first solvent may be toluene, xylene, benzene, hexane, cyclohexane, heptane, octane, dichloromethane, chloroform, dichloroethane, dimethylbenzene, or a combination thereof. According to embodiments of the disclosure, the second solvent may be methanol, ethanol, isopropanol, n-propanol, acetone, methyl ethyl ketone (MEK), ethyl acetate, dimethyl carbonate, tetrahydrofuran (THF), diethyl ether, acetonitrile, or a combination thereof.

According to embodiments of the disclosure, the ratio of the weight of the second solvent to the total weight of the first solvent and water may be 1: 1 to 1: 5, and the weight ratio of the second solvent to water may be 1: 1 to 1: 8.

According to embodiments of the disclosure, after the extraction process, the second solution may undergo an adsorption process, a precipitation process, or a combination thereof. For example, after obtaining the second solution, the second solvent may be removed, causing the alkyl bisphenol to precipitate from the water. Next, the resulting product may undergo an adsorption process. According to embodiments of the disclosure, in addition to using a co-solvent precipitation process, the solubility difference of alkyl bisphenol can also be utilized by adjusting the temperature of the solvent to cause alkyl bisphenol to precipitate. According to embodiments of the disclosure, the adsorbent used in the adsorption process may be activated carbon, ion exchange resin, diatomaceous earth, aluminum oxide, or a combination thereof.

According to embodiments of the disclosure, the method for removing the second solvent from the second solution may be a low-temperature solvent removal method, such as performing reduced-pressure concentration at 25°C to 65°C or vacuum drying at 25°C to 65°C.

Below, exemplary embodiments will be described in detail with reference to the accompanying drawings so as to be easily realized by a person having ordinary knowledge in the art. The inventive concept may be embodied in various forms without being limited to the exemplary embodiments set forth herein. Descriptions of well-known parts are omitted for clarity, and like reference numerals refer to like elements throughout.

### Example 1

Depolymerization process: 40g of polycarbonate waste flakes (sourced from polycarbonate carrier waste), 200g of dimethyl carbonate, 60g of methanol, and 1.4g of sodium hydroxide were added into a reaction bottle. Next, the reaction bottle was heated to a temperature between 60°C and 70°C and reacted for 5 hours. After the reaction bottle was cooled down to room temperature, a mixture containing alkyl bisphenol was obtained.

Extraction/precipitation process: The mixture containing alkyl bisphenol was subjected to a reduced pressure concentration using a rotary evaporator at 25°C to remove the solvent, thereby obtaining a crude alkyl bisphenol product. Next, 140g of toluene (served as the first solvent) and 60g of methanol (served as the second solvent) were added to the crude alkyl bisphenol product. After mixing, a homogeneous solution was obtained. Next, 80g of water was added to the homogeneous solution. After mixing, a two-layer solution (including the first solution and the second solution) was obtained. Next, the aqueous layer (i.e., the second solution) in the two-layer solution was collected. Next, the aqueous layer was subjected to reduced-pressure concentration at 25°C using a rotary evaporator to remove methanol. Next, the result was allowed to stand for 10 mins, during which white crystals were observed to precipitate from the water. The precipitate was collected by filtration and then vacuum-dried at 25°C to obtain white crystal (33.5g).

Adsorption/precipitation/drying process: The obtained white crystal was dissolved in 120g of methanol, and 4g of activated carbon was added. After stirring for 2 hours, the activated carbon was removed by filtration. The filtrate was then slowly added into water (1,200g), during which white crystals were observed to precipitate from the water. The precipitate was collected by filtration, vacuum-dried at 25°C for 1 hour, and then vacuum-dried at 40°C for another 1 hour to obtain regenerated Alkyl Bisphenol A composition (1)(31.6g).

Next, the APHA color of Alkyl Bisphenol A composition (1) was evaluated, and the results are shown in Table 1. Alkyl Bisphenol A composition (1) was analyzed using high performance liquid chromatography (HPLC), with a mixed solvent of acetonitrile and methanol (in a volume ratio of 9:1) and water as the mobile phase. The volume ratio of the mixed solvent to water was gradually adjusted during the analysis: initially 3:7 and changing to 9:1 after 30 minutes. The HPLC sample injection volume was 10µL, and the mobile phase flow rate was 1.0 mL/min. The results are shown in Table 1. The HPLC sample was prepared as follows. The Alkyl Bisphenol A composition (0.1g), acetonitrile (1.7g), and an o-cresol solution (0.2g) (o-cresol dissolved in acetonitrile at a concentration of 1 wt%) were mixed, then filtered through a polytetrafluoroethylene (PTFE) membrane filter with a pore size of 0.22µm to obtain the HPLC sample. The APHA color was measured by a method according to ASTM D1209-05 by dissolving 5g of the sample in 7ml of methanol for testing.

### Example 2

Example 2 was performed in the same manner as the method for preparing Alkyl Bisphenol A composition (1) disclosed in Example 1, except for the following differences. In the depolymerization process, 70g of ethanol was used instead of 60g of methanol. In the extraction/precipitation process, 140g of hexane was used instead of 140g of toluene as the first solvent, 210g of ethanol was used instead of 60g of methanol as the second solvent, and the amount of water was increased from 80g to 350g. Next, the APHA color of Alkyl Bisphenol A composition (2) (30.52g) was evaluated, and the results are shown in Table 1. Alkyl Bisphenol A composition (2) was analyzed using high performance liquid chromatography (HPLC), and the results are shown in Table 1.

### Example 3

Depolymerization process: 40g of polycarbonate waste flakes (sourced from polycarbonate carrier waste), 120g of methyl ethyl ketone (MEK), 120g of methanol, and 1.4g of sodium hydroxide were added into a reaction bottle. Next, the reaction bottle was heated to a temperature between 60°C and 70°C and reacted for 5 hours. After the reaction bottle was cooled down to room temperature, a mixture containing alkyl bisphenol was obtained.

Extraction/precipitation process: The mixture containing alkyl bisphenol was subjected to a reduced pressure concentration using a rotary evaporator at 25°C to remove the solvent, obtaining a crude alkyl bisphenol product. Next, 100g of dichloromethane (used as the first solvent) and 60g of acetone (used as the second solvent) were added to the crude alkyl bisphenol product and mixed to obtain a homogeneous solution. Next, 80g of water was added to the homogeneous solution. After mixing, a two-layer solution (including the first solution and the second solution) was obtained. Next, the aqueous layer (i.e., the second solution) of the two-layer solution was collected. Next, the aqueous layer was subjected to a reduced-pressure concentration using a rotary evaporator at 25°C to remove acetone. Next, the result was allowed to stand for 10 mins, during which white crystals were observed to precipitate from the water. The precipitate was collected by filtration and then vacuum-dried at 25°C to obtain white crystal.

Drying process: The white crystal was vacuum dried at 25°C for 1 hour and then vacuum dried at 40°C for 1 hour to obtain regenerated Alkyl Bisphenol A composition (3)(30.88g).

Next, the APHA color of Alkyl Bisphenol A composition (3) was evaluated, and the results are shown in Table 1. Alkyl Bisphenol A composition (3) was analyzed using high performance liquid chromatography (HPLC), and the results are shown in Table 1.

### Example 4

Example 4 was performed in the same manner as the method for preparing Alkyl Bisphenol A composition (1) disclosed in Example 1, except for the following differences. In the depolymerization process, 140g of ethanol and 20g of dichloromethane were used instead of 60g of methanol and 200g of dimethyl carbonate. In the extraction/precipitation process, 100g of hexane was used (as the first solvent) instead of 140g of toluene, and 60g of ethyl acetate was used (as the second solvent) instead of 60g of methanol. Next, the APHA color of Alkyl Bisphenol A composition (4) (29.45g) was evaluated, and the results are shown in Table 1. Alkyl Bisphenol A composition (4) was analyzed using high performance liquid chromatography (HPLC), and the results are shown in Table 1.

### Example 5

Example 5 was performed in the same manner as the method for preparing Alkyl Bisphenol A composition (3) disclosed in Example 3, except for the following differences. In the depolymerization process, 120g of dimethyl carbonate was used instead of 120g of methyl ethyl ketone. In the extraction/precipitation process, 70g of toluene was used (as the first solvent) instead of 100g of dichloromethane, and 350g of ethyl acetate was used (as the second solvent) instead of 60g of acetone and the amount of water was increased from 80g to 350g. Next, the APHA color of Alkyl Bisphenol A composition (5) (28.73g) was evaluated, and the results are shown in Table 1. Alkyl Bisphenol A composition (5) was analyzed using high performance liquid chromatography (HPLC), and the results are shown in Table 1.

### Example 6

Example 6 was performed in the same manner as the method for preparing Alkyl Bisphenol A composition (1) disclosed in Example 1, except that the amount of toluene was reduced from 140g to 100g, and 60g of ethyl acetate was used (as the second solvent) instead of 60g of methanol, and the amount of water was increased from 80g to 100g in the extraction/precipitation process. Next, the APHA color of Alkyl Bisphenol A composition (6) (25.86g) was evaluated, and the results are shown in Table 1. Alkyl Bisphenol A composition (6) was analyzed using high performance liquid chromatography (HPLC), and the results are shown in Table 1.

### Example 7

Example 7 was performed in the same manner as the method for preparing Alkyl Bisphenol A composition (1) disclosed in Example 1, except for the following differences. In the depolymerization process, 60g of ethanol was used instead of 60g of methanol. In the extraction/precipitation process, 70g of dimethylbenzene was used (as the first solvent) instead of 140g of toluene, 140g of ethanol was used (as the second solvent) instead of 60g of methanol, and the amount of water was increased from 80g to 280g. Next, the APHA color of Alkyl Bisphenol A composition (7) (20.14g) was evaluated, and the results are shown in Table 1. Alkyl Bisphenol A composition (7) was analyzed using high performance liquid chromatography (HPLC), and the results are shown in Table 1.

### Example 8

Example 8 was performed in the same manner as the method for preparing Alkyl Bisphenol A composition (1) disclosed in Example 1, except for the following differences. In the depolymerization process, the amount of methanol was increased from 60g to 140g, and 20g of dichloromethane was used instead of 200g of dimethyl carbonate. In the extraction/precipitation process, 100g of dimethylbenzene was used (as the first solvent) instead of 140g of toluene. Next, the APHA color of Alkyl Bisphenol A composition (8) (28g) was evaluated, and the results are shown in Table 1. Alkyl Bisphenol A composition (8) was analyzed using high performance liquid chromatography (HPLC), and the results are shown in Table 1.

**Table 1**

| | Alkyl Bisphenol A Composition | | | | APHA color |
|---|---|---|---|---|---|
| | alkyl bisphenol A (wt%) | *¹ monophenolic compound (wt%) | *² reaction adduct of alkyl bisphenol (wt%) | *³ aromatic compounds-additive or impurity derived (wt%) | |
| Example 1 /Alkyl Bisphenol A composition (1) | >99.86 | <0.01 | <0.01 | <0.12 | 15 |
| Example 2 /Alkyl Bisphenol A composition (2) | >99.93 | <0.01 | <0.01 | <0.05 | 53 |
| Example 3 /Alkyl Bisphenol A composition (3) | >99.92 | <0.01 | <0.01 | <0.06 | 22 |
| Example 4 /Alkyl Bisphenol A composition (4) | >99.89 | <0.01 | <0.01 | 0.09 | 29 |
| Example 5 /Alkyl Bisphenol A composition (5) | >99.85 | <0.01 | <0.01 | 0.13 | 52 |
| Example 6 /Alkyl Bisphenol A composition (6) | >99.92 | <0.01 | <0.01 | 0.06 | 11 |
| Example 7 /Alkyl Bisphenol A composition (7) | >99.90 | <0.01 | <0.01 | 0.08 | 24 |
| Example 8 /Alkyl Bisphenol A composition (8) | >99.91 | <0.01 | <0.01 | 0.07 | 14 |
| *¹: The monophenolic compound is phenol, cresol, xylenol. | | | | | |
| *²: The reaction adduct of bisphenol A refers to the oligomers generated during the depolymerization of polycarbonate, or the reaction product of bisphenol A and an alkyl carbonate. The reaction adduct of bisphenol A may have a structure of 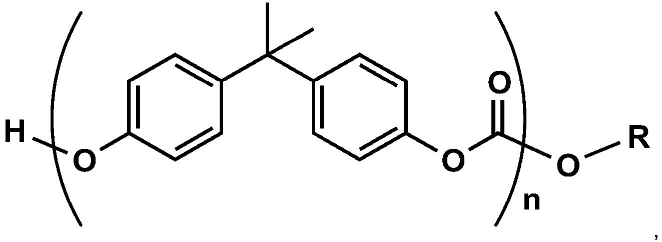 wherein R is C1-C4 alkyl group, n≥1. | | | | | |
| *³: The additive refers to an anthraquinone compound, azo compound, benzotriazole compound, or benzopyran compound used in polycarbonate products (serving as a flame retardant, dye, UV absorber, mold release agent, or antioxidant). The impurities derived from additives refer to additive reaction adducts formed by side reactions during the preparation of the bisphenol A composition. | | | | | |

As shown in Table 1, through the specific polycarbonate waste recycling process of the disclosure (including specific extraction steps involving the simultaneous use of a first solvent, second solvent, and water), the regenerated bisphenol A composition prepared according to the disclosure can achieve a bisphenol A content greater than 99.85 wt%, a monophenolic compound content less than 0.01 wt%, a reaction adduct of bisphenol A content less than 0.01 wt%, and an aromatic compound (additives or impurities derived therefrom) content less than 0.13 wt%. In addition, the APHA color value of the regenerated bisphenol A composition of the disclosure is less than 55, and the purification process yield is favorable (for example, the purification process yield of Alkyl Bisphenol A composition (1) in Example 1 is approximately 88%).

### Comparative Example 1

Depolymerization process: 40g of polycarbonate waste flakes (sourced from polycarbonate carrier waste), 200g of dimethyl carbonate, 60g of methanol, and 1.4g of sodium hydroxide were added into a reaction bottle. Next, the reaction bottle was heated to a temperature between 60°C and 70°C and reacted for 5 hours. After the reaction bottle was cooled down to room temperature, a mixture containing alkyl bisphenol was obtained.

Extraction process: The mixture containing alkyl bisphenol was subjected to a reduced-pressure concentration using a rotary evaporator at 25°C to remove the solvent, obtaining a crude alkyl bisphenol product. Next, 50g of hexane (used as the first solvent) and 100g of ethyl acetate (used as the second solvent) were added to the crude alkyl bisphenol product and mixed to obtain a non-homogeneous solution. Next, the ethyl acetate extraction layer in the non-homogeneous solution was collected. Next, the result was subjected to reduce-pressure concentration at 25°C using a rotary evaporator to remove ethyl acetate, obtaining a white solid.

Precipitation/drying process: The obtained white solid was dissolved in methanol (120g), and the resulting solution was slowly added to water (1,200g), during which white crystals were precipitated from the water. The precipitate was collected by filtration, vacuum-dried at 25°C for 1 hour, and then vacuum-dried at 40°C for 1 hour to obtain Alkyl Bisphenol A composition (9) (25.9 g).

Next, the APHA color of Alkyl Bisphenol A composition (9) was evaluated, and the results are shown in Table 2. Alkyl Bisphenol A composition (9) was analyzed using high performance liquid chromatography (HPLC), and the results are shown in Table 2.

### Comparative Example 2

Depolymerization process: 40g of polycarbonate waste flakes (sourced from polycarbonate carrier waste), 200g of dimethyl carbonate, 60g of methanol, and 1.4g of sodium hydroxide were added into a reaction bottle. Next, the reaction bottle was heated to a temperature between 60°C and 70°C and reacted for 5 hours. After the reaction bottle was cooled down to room temperature, a mixture containing alkyl bisphenol was obtained.

Washing process: The mixture containing alkyl bisphenol was subjected to reduce-pressure concentration at 25°C using a rotary evaporator to remove the solvent and obtain a crude alkyl bisphenol product. Next, the crude alkyl bisphenol product was added to toluene (100 g) and mixed to obtain a first solution. The first solution was then heated to 80°C and subsequently cooled slowly down to 25°C. After standing for 1 hour, the precipitate was collected by filtration to obtain a white solid.

Reductant addition process: The obtained white solid was mixed with 50g of methanol and 50g of water, followed by the addition of sodium dithionite (Na₂S₂O₄) (0.06 g). The mixture was stirred to obtain a second solution.

Adsorption/drying process: 10g of activated carbon was added to the second solution. After stirring for 2 hours, the activated carbon was removed by filtration. Next, the solvent of the filtrate was removed at 25°C using a rotary evaporator. The result was then vacuum-dried at 25°C for 1 hour and subsequently vacuum-dried at 40°C for 1 hour to obtain Alkyl Bisphenol A composition (10) (30.9g).

Next, the APHA color of Alkyl Bisphenol A composition (10) was evaluated, and the results are shown in Table 2. Alkyl Bisphenol A composition (10) was analyzed using high performance liquid chromatography (HPLC), and the results are shown in Table 2.

### Comparative Example 3

Depolymerization process: 40g of polycarbonate waste flakes (sourced from polycarbonate carrier waste), 200g of dimethyl carbonate, 60g of methanol, and 1.4g of sodium hydroxide were added into a reaction bottle. Next, the reaction bottle was heated to a temperature between 60°C and 70°C and reacted for 5 hours. After the reaction bottle was cooled to room temperature, a mixture containing alkyl bisphenol was obtained.

Adsorption/precipitation/drying process: The mixture containing alkyl bisphenol was subjected to a reduced pressure concentration at 25°C using a rotary evaporator to remove the solvent and obtain a crude alkyl bisphenol product. Next, the crude alkyl bisphenol product was dissolved in methanol (120g), followed by the addition of 12g of activated carbon. After stirring for 2 hours, the activated carbon was removed by filtration, and the filtrate was slowly added into water (1,200g), during which white crystals were precipitated from the water. The precipitate was collected by filtration, vacuum-dried at 25°C for 1 hour, and then vacuum-dried at 40°C for 1 hour to obtain Alkyl Bisphenol A composition (11)(8.85g).

Next, the APHA color of Alkyl Bisphenol A composition (11) was evaluated, and the results are shown in Table 2. Alkyl Bisphenol A composition (11) was analyzed using high performance liquid chromatography (HPLC), and the results are shown in Table 2.

### Comparative Example 4 (according to US20230322653A1)

Depolymerization process: 40g of polycarbonate waste flakes (sourced from polycarbonate carrier waste), 200g of dimethyl carbonate, 60g of methanol, and 1.4g of sodium hydroxide were added into a reaction bottle. Next, the reaction bottle was heated to a temperature between 60°C and 70°C and reacted for 5 hours. After the reaction bottle was cooled to room temperature, a mixture containing alkyl bisphenol was obtained.

Washing/drying process: The mixture containing alkyl bisphenol was subjected to a reduced pressure concentration at 25°C using a rotary evaporator to remove the solvent and obtain a crude alkyl bisphenol product. Next, the crude alkyl bisphenol product was added to toluene (100 g) and mixed to obtain a first solution. Next, the first solution was then heated to 80°C, followed by washing with water five times (100g of water each time), and the organic phase was collected. The organic phase was then slowly cooled to 25°C. After standing for 1 hour, the precipitate was collected by filtration to obtain a white solid. The white solid was then vacuum-dried at 25°C for 1 hour and at 40°C for 1 hour to obtain Alkyl Bisphenol A composition (12) (28g).

Next, the APHA color of Alkyl Bisphenol A composition (12) was evaluated, and the results are shown in Table 2. Alkyl Bisphenol A composition (12) was analyzed using high performance liquid chromatography (HPLC), and the results are shown in Table 2.

### Comparative Example 5 (according to US20230391702A1)

Depolymerization process: Polycarbonate waste (41.5g) and dichloromethane (226.9g) were added to a reaction bottle. After mixing, the polycarbonate waste was dissolved in dichloromethane. Next, methanol (42g), ethanol (72.35g), and sodium hydroxide (2.1g) were added to the reaction bottle. The reaction bottle was then heated to 60°C and reacted for 6 hours. After the reaction bottle was cooled to room temperature, a mixture containing alkyl bisphenol was obtained.

Adsorption process: Next, the mixture containing alkyl bisphenol was cooled down to 20°C, and an aqueous hydrochloric acid (HCl) solution (10g, with a concentration of 1N) was added to the mixture containing alkyl bisphenol to obtain a solution. Next, 12.45g of activated carbon was added to the solution. After stirring for 2 hours, the activated carbon was removed by filtration, and the filtrate was subjected to a reduced pressure concentration at 25°C using a rotary evaporator to remove the solvent.

Washing/adsorption/precipitation/drying process: Next, the result was washed with dichloromethane (41.5g) at 20°C. After filtration, the filtrate was washed with water (124.5g) at 50°C, repeated twice. Next, 80.5g of ethanol was added to the resulting solution. Next, 12.45g of activated carbon was added to the solution. After stirring for 2 hours, the activated carbon was removed by filtration. Next, water (1,000g) was mixed with the filtrate and stirred. After standing for 10 minutes, white crystals were precipitated from the water. The precipitate was collected by filtration and dried in a vacuum oven at 30°C to obtain Alkyl Bisphenol A composition (13) (4.71 g).

Next, the APHA color of Alkyl Bisphenol A composition (13) was evaluated, and the results are shown in Table 2. Alkyl Bisphenol A composition (13) was analyzed using high performance liquid chromatography (HPLC), and the results are shown in Table 2.

**Table 2**

| | Alkyl Bisphenol A Composition | | | | APHA color | purification yield (%) |
|---|---|---|---|---|---|---|
| | alkyl bisphenol A (wt%) | monophenolic compound (wt%) | reaction adduct of bisphenol A (wt%) | additives or impurities derived from the additives (wt%) | | |
| Example 1 /Alkyl Bisphenol A composition (1) | >99.86 | <0.01 | <0.01 | <0.12 | 15 | 88% |
| Comparative Example 1 /Alkyl Bisphenol A composition (9) | 95.43 | 0.01 | 3.79 | 0.77 | 373 | (with a purity of less than 99%) |
| Comparative Example 2 /Alkyl Bisphenol A composition (10) | 98.56 | 0.03 | 1.36 | 0.05 | 323 | (with a purity of less than 99%) |
| Comparative Example 3/Alkyl Bisphenol A composition (11) | 98.1 | 0.01 | 1.06 | 0.83 | 375 | (with a purity of less than 99%) |
| Comparative Example 4 /Alkyl Bisphenol A composition (12) | 95.66 | 0.01 | 0.05 | 4.28 | 300 | (with a purity of less than 99%) |
| Comparative Example 5 /Alkyl Bisphenol A composition (13) | 98.53 | 0.01 | 0.02 | 1.44 | >300 | 10% (with a purity of less than 99%) |

Comparative Example 1 did not utilize the extraction process (simultaneously using a first solvent/second solvent/water) and adsorption process described in Example 1. As shown in Table 2, Alkyl Bisphenol A composition (9) obtained in Comparative Example 1 had a significantly higher content of reaction adduct of bisphenol A (>3 wt%) and additives (or impurities derived from the additives) (>0.7 wt%), and its APHA color value was also greater than 300.

Comparative Example 2 did not utilize the extraction process (simultaneously using a first solvent/second solvent/water) described in Example 1. As shown in Table 2, even though additional washing and reductant addition processes were applied, Alkyl Bisphenol A composition (10) obtained in Comparative Example 2 still had significantly higher levels of monophenolic compound (about 0.03 wt%), reaction adduct of bisphenol A (>1 wt%), and additives (or impurities derived therefrom) (about 0.05 wt%), and its APHA color value also exceeded 300.

Comparative Example 3 did not utilize the extraction process (simultaneously using a first solvent/second solvent/water) described in Example 1. As shown in Table 2, despite undergoing adsorption, precipitation, and drying processes, Alkyl Bisphenol A composition (11) obtained in Comparative Example 3 still showed a relatively high content of reaction adduct of bisphenol A (about 0.05 wt%>1 wt%) and additives (or impurities derived therefrom) (>0.8wt%), with an APHA color value also greater than 300.

Comparative Example 4 did not utilize the extraction process (simultaneously using a first solvent/second solvent/water) described in Example 1. As shown in Table 2, although the purification process followed the method disclosed in US20230322653A1, Alkyl Bisphenol A composition (12) obtained in Comparative Example 4 still had a significantly higher content of reaction adduct of bisphenol A (about 0.05 wt%) and additives (or impurities derived therefrom) (>4 wt%), with an APHA color value achieved about 300.

Comparative Example 5 also did not utilize the extraction process (simultaneously using a first solvent/second solvent/water) described in Example 1. As shown in Table 2, although the depolymerization and purification processes followed the method disclosed in US20230391702A1, Alkyl Bisphenol A composition (13) obtained in Comparative Example 5 still exhibited a significantly high level of additives (or impurities derived therefrom) (>1 wt%), an APHA color value greater than 300, and a low purification yield of less than 10% after being subjected to adsorption processes twice.

Accordingly, due to the specific multi-solvent extraction process, the method for preparing the alkyl bisphenol composition of the disclosure can significantly reduce the impurity content in the alkyl bisphenol composition without compromising the recovery yield. This allows for the production of regenerated alkyl bisphenol compositions with high purity (greater than or equal to 99.85%) and low APHA color value (less than or equal to 80), which can enhance the quality of regenerated polycarbonate products.

It will be clear that various modifications and variations can be made to the disclosed methods and materials. It is intended that the specification and examples be considered as exemplary only, with the true scope of the disclosure being indicated by the following claims and their equivalents.

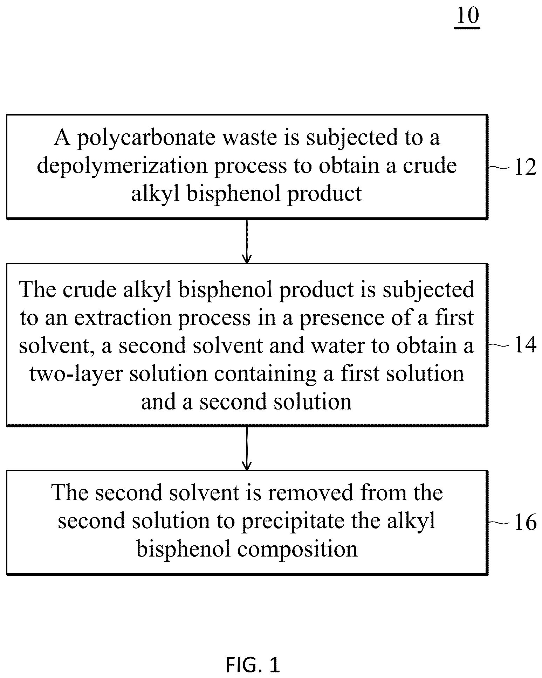

## Claims

1. An alkyl bisphenol composition, comprising:
99.85wt% to 99.999wt% of an alkyl bisphenol;
0.0001wt% to 0.01wt% of a monophenolic compound;
0.0001wt% to 0.01wt% of a reaction adduct of alkyl bisphenol; and
0.0001wt% to 0.1497wt% of an aromatic compound, wherein the aromatic compound is not the alkyl bisphenol, the monophenolic compound, or the reaction adduct of alkyl bisphenol.

2. The alkyl bisphenol composition as claimed in Claim 1, wherein the alkyl bisphenol has a structure represented by Formula (I): 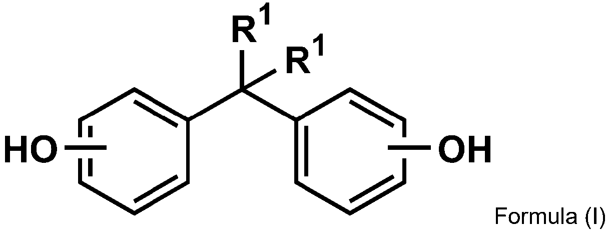 , wherein each R1 is independently C1-C4 alkyl group.

3. The alkyl bisphenol composition as claimed in Claim 1, wherein the monophenolic compound is phenol, cresol, xylenol, or a combination thereof.

4. The alkyl bisphenol composition as claimed in Claim 1, wherein the reaction adduct of alkyl bisphenol has a structure represented by Formula (II): 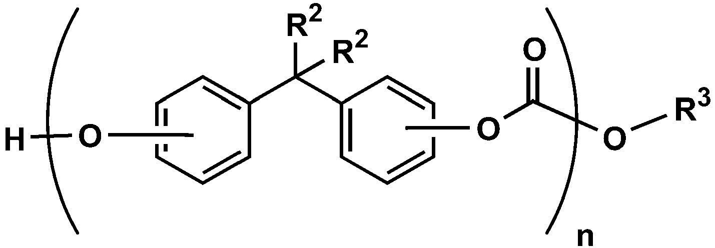 , wherein R2 and R3 are independently C1-C4 alkyl group; and, n≥1.

5. The alkyl bisphenol composition as claimed in Claim 1, wherein the aromatic compound is an anthraquinone compound, an azo compound, a benzotriazole compound, a benzopyran compound, or a combination thereof.

6. A method for preparing an alkyl bisphenol composition, comprising:
subjecting a polycarbonate waste to a depolymerization process to obtain a crude alkyl bisphenol product;
subjecting the crude alkyl bisphenol product to an extraction process in presence of a first solvent, a second solvent and water to obtain a two-layer solution containing a first solution and a second solution, wherein the first solution comprises the first solvent, and the second solution comprises the second solvent, water, and alkyl bisphenol, wherein the first solvent is not miscible with water, and the second solvent is miscible with water; and
removing the second solvent from the second solution to precipitate the alkyl bisphenol composition as claimed in Claim 1 from the second solution.

7. The method for preparing an alkyl bisphenol composition as claimed in Claim 6, further comprising:
subjecting the second solution to an adsorption process, a precipitation process, or a combination thereof, after the extraction process.

8. The method for preparing an alkyl bisphenol composition as claimed in Claim 6, wherein in the extraction process, the first solvent and the second solvent are first mixed with the crude alkyl bisphenol product, and then extracted with water.

9. The method for preparing an alkyl bisphenol composition as claimed in Claim 6, wherein in the extraction process, the second solvent and water are first mixed with the crude alkyl bisphenol product, and then extracted with the first solvent.

10. The method for preparing an alkyl bisphenol composition as claimed in Claim 6, wherein in the extraction process, the second solvent is first mixed with the crude alkyl bisphenol product, and then extracted with the first solvent and water.

11. The method for preparing an alkyl bisphenol composition as claimed in Claim 6, wherein the first solvent is toluene, xylene, benzene, hexane, cyclohexane, heptane, octane, dichloromethane, chloroform, dichloroethane, dimethylbenzene, or a combination thereof.

12. The method for preparing an alkyl bisphenol composition as claimed in Claim 6, wherein the second solvent is methanol, ethanol, isopropanol, n-propanol, acetone, methyl ethyl ketone, ethyl acetate, dimethyl carbonate, tetrahydrofuran, diethyl ether, acetonitrile, or a combination thereof.

13. The method for preparing an alkyl bisphenol composition as claimed in Claim 6, wherein the polycarbonate waste is subjected to a depolymerization process in the presence of a depolymerization composition, wherein the depolymerization composition comprises a C1-C6 aliphatic monoalcohol.

14. The method for preparing an alkyl bisphenol composition as claimed in Claim 13, wherein the depolymerization composition further comprises a co-solvent, wherein the co-solvent is dichloromethane, chloroform, carbon tetrachloride, cyclohexanone, methyl ethyl ketone, acetone, toluene, xylene, dimethyl carbonate, diethyl carbonate, or a combination thereof.

15. The method for preparing an alkyl bisphenol composition as claimed in Claim 13, wherein the depolymerization composition further comprises a catalyst, wherein the catalyst is sodium hydroxide, potassium hydroxide, or a combination thereof.
